# EUROPEAN PATENT SPECIFICATION

(11) **EP 1 677 127 B1**
(45) Date of publication and mention of the grant of the patent: **03.09.2008**
(21) Application number: 04425955.4
(22) Date of filing: 29.12.2004
(51) Int. Cl.: G01S 15/89, G01S 7/52, A61B 8/08, G06T 1/00

(54) **Method and system for processing digital ultrasonic image sequences**
Verfahren und System zur Verarbeitung digitaler Ultraschall-Bildsequenzen
Procédé et système de traitement de séquences d'images ultrasonores numériques

(43) Date of publication of application: 05.07.2006
(73) Proprietor: eMedStream S.r.l., 20129 Milano (IT)
(72) Inventor: Barbier, Paolo, 20052 Monza (IT)
(74) Representative: Siniscalco, Fabio

(56) References cited:
- EP-A- 1 107 012
- EP-A- 1 482 329
- US-A- 4 271 842
- US-A- 4 572 202
- US-A- 5 619 995
- US-A- 5 976 088
- US-B1- 6 231 510
- US-B1- 6 350 238

## Description

The present invention refers to a method and system for processing digital images. Particularly, but not limitatively, the present invention refers to the processing of images acquired with equipment used for clinical examinations, such as, for example, equipment based on ultrasound techniques.

A clinical test technique widely used in the medical field, known with the name of "echography", is characterized by the detection of ultrasonic waves reflected by the tissues of a living being. Such technique consists in the sending of beams of ultrasonic pulses (with a frequency between 2 and 20 MHz) at regions of the body subject to the clinical tests. Such pulse beams are sent by means of an appropriate echographic equipment provided with a probe that has to be placed and moved on the surface of the body.

The ultrasonic pulses, penetrating the human living body, undergo reflections at the border surfaces between different type of tissues and on the walls of the hollow organs. The reflected pulses (or echoes) are received by the same probe and transformed into electric pulses successively visualized as images on a screen of the echographic equipment.

The acquired ultrasonographic images can be presented in a static or dynamic form. In the latter case, using multiple rapid scans of the part of the body under investigation by means of ultrasonic pulses, it is possible to obtain an image sequence or video sequence of the internal organs of the body.

In the majority of medical centers, the diagnostic ultrasonographic images corresponding to each echographic exam are stored using video tape recording (Video Cassette Recorder or VCR).

However, such means of analogical storing on video tapes produces an immediate reduction of the quality of the recorded images with respect to the originally acquired images. In addition, the retrieval of the stored images is not practical due to the sequence nature of the recording on the tape.

Furthermore, it could be difficult to compare exams carried out on the same individual at different times or images obtained at different times during the same exam with consequent decline of the diagnostic accuracy.

With the spread of digital technology, the analogical video sequences obtained with the ultrasonographic equipment can be digitalized in order to be stored. In fact, as known, the stored digital images are accessible in a random way and the same can be reproduced for consultation maintaining unaltered the original quality.

To this end, a DICOM (Digital Imaging and Communication in Medicine) standard was introduced to uniform the digital format of the acquired diagnostic images and the development of PACS (Picture Archiving and Communication System) filing system was promoted for the creation of a searchable database containing the abovementioned image sequences.

When using an ultrasonographic equipment in digital modality, an operator selects from the entire video sequence relative to an echographic examination only some sub-sequences considered significant for diagnostic purposes. For example, for an examination with a 15-20 minutes duration, about 20 sub-sequences (or video clip) are acquired, each one having a variable duration from one to three seconds, as well as ten still frame images.

This selection represents the known "clinical compression" carried out mostly for the storing of the images. According to the conventional techniques, the operator "manually" sends the specific commands to an image elaboration apparatus parallel to the carrying out of the echographic examination which, as already said, implies the manual handling of the probe.

The modality with which the clinical compression occurs in conventional apparatus is uncomfortable and such as to interfere with the attention of the operator towards the diagnostic process.

The video clips (each one characterized by a rapid succession of photograms or frames) and the fixed images obtained by clinical compression are further compressed by a video compression algorithm in order to be stored. For example, the algorithm used is MPEG-2 (Motion Pictures Expert Group) which was validated in the medical field inasmuch as the quality of the compressed video clips and of the same decompressed, in order to be visualized, assures an accuracy consistent with the medical diagnosis. As known, after compression and decompression, the video clips lose some information compared to the original non compressed video clips. For this reason the MPEG-2 algorithm is said to be "lossy".

Document US 6,231,510 B1 discloses a digital video recorder which provides digital storage of realtime ultrasonic image sequences replacing the conventional VCR with a recorder which does not degrade digital ultrasound images by analog conversion and recording.

The object of the present invention is that of proposing a method of digital imaging processing, in particular for diagnostic reasons, which does not present the abovementioned disadvantages of the present existing solutions.

This object is reached by a processing method as defined in the attached Claim 1. Preferred embodiments of the method of the invention are defined by the dependent Claims from 2 to 16.

The object of the present invention is also an apparatus defined in Claim 17 and a digital images processing system as defined in Claims 18 and 19.

The characteristics and the advantages of the present invention will be understood from the following detailed description of an exemplary and non-limiting embodiment thereof, with reference to the annexed drawings, in which:
- figure 1 schematically shows a processing and transmission method of ultrasonographic images in accordance with the invention;
- figure 2A schematically shows a few processing blocks of the system show in figure 1;
- figure 2B schematically shows software modules implemented in a service processor or server used in figure 1.

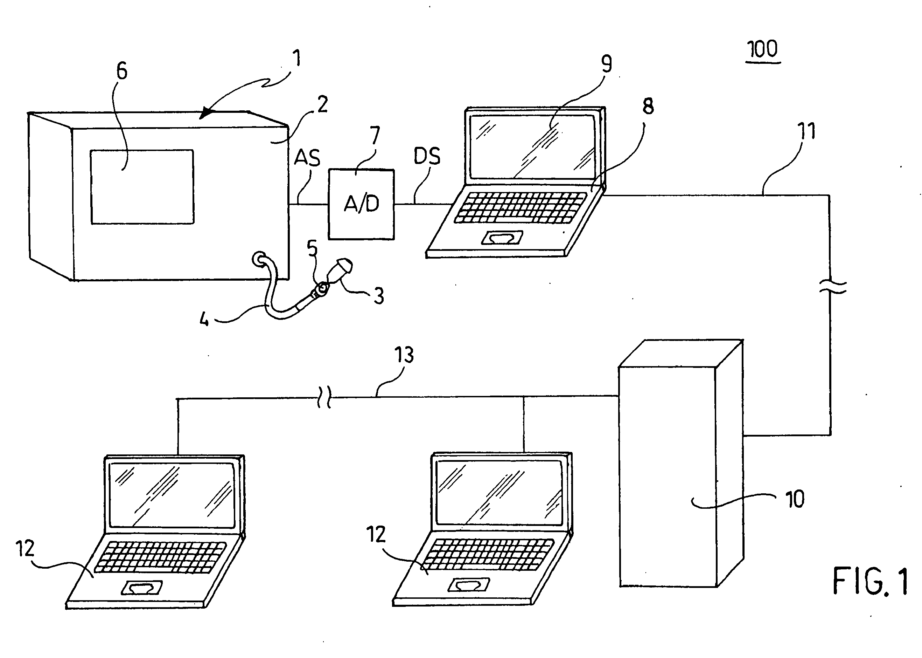

A system 100 for processing and remote transmitting digital images in accordance with an example of the invention is shown, schematically, in figure 1.

In particular, such digital images relate to a clinical examination, are used for medical-diagnostic reasons and, for example, are obtained with an ultrasound technique.

According to the provided example, the system 100 includes an equipment 1 to acquire a sequence of images relative to a clinical examination carried out with ultrasound technique, that is, an echographic equipment or echograph. Generally, such equipment 1, of the conventional type, includes a main block 2 to carry out clinical examination management functions (or echographic examination) and a probe 3 having a tip 5 connected to the main block 2 by means of an electric cable 4.

In more detail, the main block 2 can include internally a plurality of circuital modules, that is hardware components (for example, microprocessors, amplifiers, memories, etc.) and software modules, that is a sequence of program instructions.

Furthermore, the probe 3 includes one or more piezoelectric transducers (not shown in figure 1), generally of the ceramic type, to generate, starting from appropriate electric signals, ultrasound beams or exploring beams to be sent to a human body on which the probe 3 is positioned.

Further ultrasound beams generated by reflection of the exploring beams through the body tissues are intercepted as "echoes" by the probe 3 and converted by the transducers in further electric signals. The processing of such reflected electric signals within the main block 2 (for example, by a microprocessor) determines the formation of images of the internal structures of the body on a first video screen 6 of the equipment 1.

For example, in a visualization modality on the first screen 6 of the reflected echoes, denominated brightness image or B-mode image, at every reflected echo corresponds a luminous point reproduced on the screen 6 (corresponding to a pixel if such screen 6 is digital). Following an electronic processing of the various luminous points it is possible to obtain a bidimensional reproduction of the internal structures of the body to identify both the normal organs and a vast range of possible pathological alterations.

As known to those skilled in the art, the echographic equipment 1 can also include devices that can evidence variations of frequency (by the Doppler effect) of the reflected echoes from moving tissues, relative to the frequency of the exploring beam. In particular, such echographic equipment 1 permits to observe the direction and the velocity of the haematic flow in the blood vessels.

Furthermore, the equipment 1 can be an echotomograph in order to carry out an echographic tomography or layered scan of the human body, that is to originate ultrasonic images of the sections of the body. In this case, the probe 3 of the echotomograph includes numerous aligned transducers which are stimulated in sequence in order to send the ultrasound pulses radially along the arch of a circle. Moving such probe in correspondence of determinate regions of the body it is possible to carry out a scan of an organ along different anatomic layers.

The equipment 1 for echography (or echotomography) is equipped with an output port to supply a first analogical video signal AS indicative of the sequence of the analogical ultrasound images obtained during the clinical examination and visualized on the first screen 6. For example, such first video signal AS is in accordance to known analogical video recording standards, as, for example, the VHS standards (Video Home System) or s-VHS (super-VHS).

Furthermore, such output port is connected to an analogical/digital conversion module or converter 7 having a function of digitalizing the first incoming video signal received. The converter 7 is represented, for example, by an A/D conversion board (16 or 24 bit) to supply as output a second digital video signal DS, that is a sequence of digitalized images corresponding to the sequence acquired with the equipment 1. In particular, the second video signal DS has a Digital Video (DV) or MPEG-2 format in order to be supplied to a processing apparatus 8.

Preferably, such processing apparatus 8 is realized by a portable personal computer (notebook) configured to acquire and process the second signal DS, that is the sequence of digitalized images. In fact, as known to those skilled in the art, a notebook includes, essentially, processing means (CPU or Central Processing Unit), digital data memorizing means (mass memory or Hard Disk, temporary volatile memories or RAM) and input ports for digital data, e.g. of the Firewire or USB types. Furthermore, the notebook 8 includes a second screen 9 (e.g. liquid crystals LCD) to visualize the sequence of images digitalized simultaneously shown also on the first video screen 6 of the equipment 1.

Preferably, the minimum requirements for the notebook 8, utilizable in the invention foresee, for example:
- a 3.0 MHz hyperthreading CPU processor;
- a 524 MB RAM memory;
- a 64 MB ATI Radeon video board;
- a Firewire IEEE 1364 digital video input;
- a screen 9 with a 1024x762 pixel resolution.

Furthermore, a service processing unit or server 10 is connectable to the notebook 8 to receive from the latter sub-sequences of the digitalized image sequences obtained following the processing of the second video signal DS. For example, the server 10 could be a portable computer (notebook) or a desktop computer.

As shown in figure 1, the connection between the notebook 8 and the server 10 is schematized through a first line 11.

It is observed that the notebook 8 and the server 10 can be positioned at a short distance with one another (e.g. within the same building of a medical echographic laboratory or in nearby buildings of a hospital), or else they can be placed in different distant locations (e.g. the notebook 8 in an echographic laboratory in one hospital and the server 10 in another hospital).

In the first case, the first line of connection 11 is, for example, a hospital Local Area Network (LAN) or else a Virtual Private Network (VPN) or whatever other type of broad band transmission line.

In the case of different locations which are distant from each other, the first line 11 can also be a narrow band line, that is a line which ensures the transfer of the data at a speed inferior to 1Mbit/sec.

More in particular, such line 11 can schematize a narrow band telecommunication system on a fixed network or radiomobile network. For example, the notebook 8 and the server 10 can be connected through an integrated digital telephone line ISDN (Integrated Services Digital Network) having a transmission speed of 128 kbit/sec. Or else, the line 11 could be a telephone line equipped with ADSL (Asymmetric Digital Subscriber Line) with a transmission speed of 256 kbit/sec or even, a simple telephone duplex cable equipped only with a 56 kbit/sec traditional modem.

Alternatively, the notebook 8 could be equipped with a wireless connection board for data transmission/reception via internet in accordance with the protocols GPRS (General Packet Radio Service) and UMTS (Universal Telecommunications System).

Furthermore, the server 10 includes a mass memory to memorize the sub-sequences of digitalized images received from the notebook 8. Such memorized sub-sequences contribute to realize a data base of digital images consultable by users qualified to access such data base. As shown in figure 1, the access to the data base of the server 10 is made through a plurality of further processing apparatuses 12 with "client" functions connected to the server 10 through a second line 13. Such further processing apparatuses 12 can be, for example, portable computers (notebook). Furthermore, the second line 13 is assimilable, for example, to a LAN line if the apparatuses 12 are located close to the server 10 (e.g. in the same building). Alternatively, the second line 13 is, for example, an ISDN telephone line or a telephone line provided with ADSL connection. Furthermore, such line 13 is assimilable , for example, to a GPRS radiomobile transmission system (or UMTS).

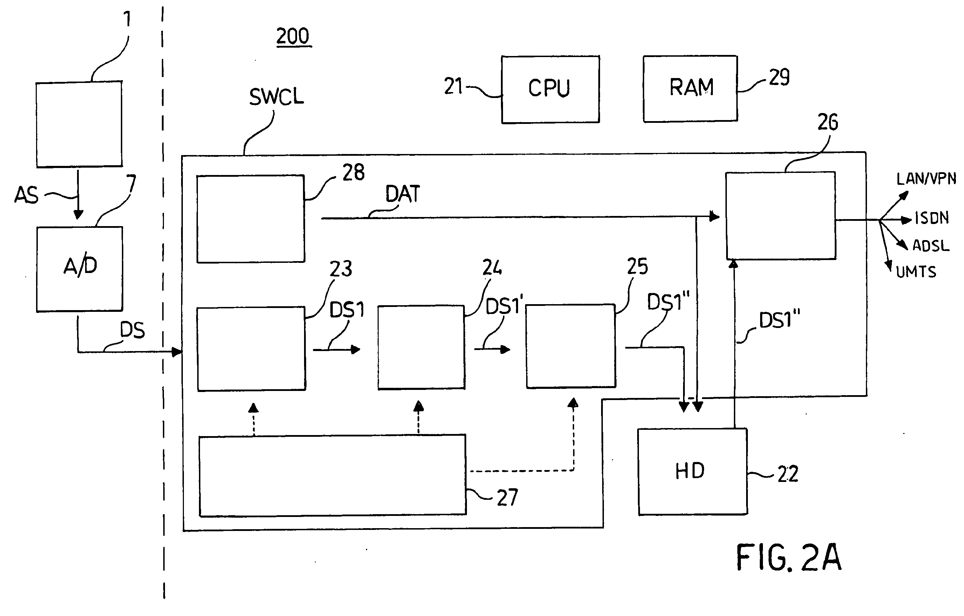

An example of the structure of the notebook 8 is shown in figure 2A. In particular, such figure 2A shows, in a schematic way, a sub-block 200 included in the notebook 8, the echographic equipment 1 and the analogical/digital conversion module 7.

In particular, it can be observed that the sub-block 200 includes both circuit modules, that is hardware components, and software modules, that is program instruction sequences. Among the circuit modules, the sub-block 200 includes a microprocessor 21 (CPU), a mass memory 22 (HD) and a temporary main memory 29 (RAM). The software modules are represented through an acquisition block 23, a cropping block 24 and a compression block 25. The blocks 23-25 are program blocks, that is they represent procedures and functions relative to a digital image sequence processing program to be sent to the server 10. In other words, such procedures and functions are indicative of processing phases to be carried out on the above mentioned images.

The blocks 23-25 are shown, altogether, with the reference SWCL, that is software client. Furthermore, the software client SWCL includes a transmission program block 26, a configuration block 27 and a data input block 28 for the management of the processing and of the transmission of the image sequences.

The software client SWCL is, for example, memorized in the mass memory 22 of the notebook 8.

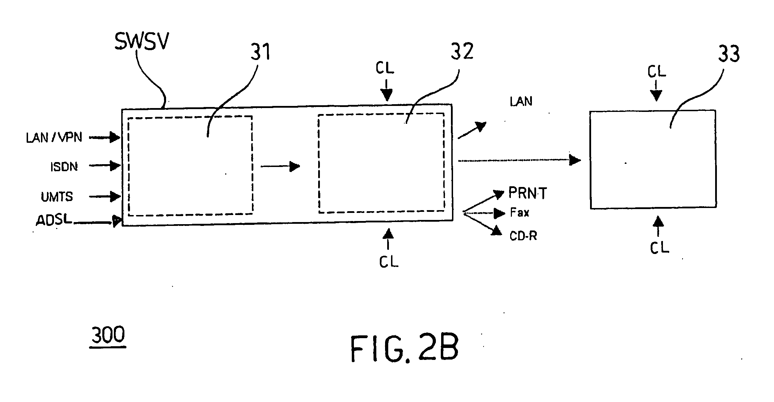

A further software module 300 implemented in the server 10 of figure 1 is shown in figure 2B. In particular, the software module 300 includes further program blocks, that is a receiving block 31 and a playback block 32 of the images sent by the notebook 8. Such blocks 31 and 32 are indicated altogether with the reference SWSV, i.e. software server.

Furthermore, the software module 300 includes a further program block 33 for the management of internet or telephone accesses (or else through another suitable telecommunications network) to the data base of the images stored in the server 10.

In relation to the figures 1, 2A and 2B a method is described, in accordance with the present invention, to process and transmit remotely the digital image sequences obtained with medical ultrasonographic equipments during clinical echographic or echotomographic examinations carried out on a living human body. Clearly reference is made to an echographic examination of the heart or echocardiographic examination, but the method is applicable also to other techniques of diagnostic investigation which foresee the acquisition of images, such as, for example, angiography.

Before the beginning of the examination or echocardiographic session, the echographic equipment 1 is connected to the analogical/digital converter 7 and to the notebook 8 by means of the analogical output port.

Furthermore, if the echocardiographic session is carried out in the same hospital in which the server 10 is located, the notebook 8 can be connected directly to the latter through the hospital LAN 11. On the contrary, that is for a server 10 situated for example in another city or in another region, the notebook 8 can be connected to a ISDN (or ADSL) telephone network. In both cases, at the beginning of the echocardiographic session, the notebook 8 is capable of automatically establishing a remote connection with the server 10.

As known, the echocardiographic examination is carried out by an operator (sonographer) who moves the probe 3 of the equipment 1 in correspondence to the parts of the living human body subject to the investigation.

Generally, such operator is a medical echographer capable of interpreting the images obtained and visualized on the first screen 6 to consequently guide the clinical examination.

During the whole echocardiographic session, the duration of which may vary, generally between ten and thirty minutes, the equipment 1 acquires in succession ultrasonographic images relative to the internal parts of the living human body. Such succession of images or sequence of images is a continuous stream or video stream sent to the A/D converter 7 as a first analogical video signal AS in VHS or s-VHS format.

In correspondence to the converter 7, the first analogical video signal AS is first digitalized and, successively, compressed using an MPEG-2 or DV codec in accordance to standard techniques known to the skilled person.

In particular, the compression carried out by such codecs generates the second video signal DS indicative of a continuous stream or video stream of digital images which are sent from the converter 7 to the notebook 8.

It is observed that the compression through the MPEG-2 or DV codec reduces the informative content of the image sequences obtained according to a ratio for example of 4 to 1. Nevertheless, it has been noted that the quantity of data relative to the digitalized image sequence from the whole echocardiographic session is considerable even after the compression carried out in the converter 7.

For example, 7-10 Mbyte of data per second are transferred to the notebook 8 with a second signal DS inasmuch as the digitalized images of the sequence are high fidelity. Consequently, in order to memorize the images relative to the whole session of 15 minutes it would be necessary, for example, from 6,3 to 9 Gbyte of memory. Due to the excessive quantity of data relative to each echocardiographic examination, in the medical field it is preferable to memorize only significant portions of the image sequences.

Furthermore, it is foreseen that the image sequence of the echocardiographic session can be supplied to the notebook 8 already in digital format (MPEG-2 or DV) through a further echographic equipment (not shown in the figure) provided with a further output port for digital signals.

Advantageously, the conversion-compression procedure of the first signal AS carried out by the converter A/D 7 is almost instantaneous, that is, it introduces a negligible temporal delay between the first signal AS and the second signal DS. In this way, the operator can observe the sequence of images of the echocardiographic session of the first video screen 6 of the equipment 1 and, simultaneously, also on the second screen 9 of the notebook 8.

In correspondence to the notebook 8, the second signal DS indicative of the digital image sequence of the echocardiographic session is processed in accordance to the operations carried out by the acquisition block 23, by the cropping block 24 and by the compression block 25 of the software client SWCL.

Such elaboration can be carried out directly by the microprocessor 21 of the notebook 8 or, alternatively, a dedicated processing unit, as for example a DSP (digital signaling processor) is foreseen.

From the observation of such ultrasonographic images acquired with the equipment 1, the operator establishes when to start the processing of the image sequence. For example, the start of the processing can correspond to the pressing of a key on the keyboard of notebook 8 predisposed for such purpose.

In a preferred embodiment of the invention, at the start of the processing, the microprocessor 21 picks up a first image sub-sequence DS1 (or video clip) of the image sequence represented by the second signal video DS, that is, the microprocessor 21 acquires a first segment of the digital image input stream.

Such phase of picking up of the first sub-sequence DS1 is carried out in accordance with the operations of the acquisition block 23 of the software client SWCL and the first sub-sequence DS1 picked up is memorized in the temporary memory 29 of the notebook 8.

It is observed that the first image sub-sequence DS1 has a temporal duration equal to a first time interval T1. For example, such first time interval T1 has a 2 seconds duration and contains about fifty digitalized images (frames). The quantity of digital information associated with the images of the first sub-sequence DS1 of 2 seconds is about 52,2 Mbyte.

Consequently, the first sub-sequence DS1 undergoes a processing phase to reduce the quantity of information necessary to define each image.

In more detail, a cropping operation of the digitalized images specified by the cropping block 24 is foreseen. Such cropping operation allows the elimination of non significant parts of the images from each image of the first sub-sequence DS1, that is, those parts having an informative content of little use for diagnostic reasons or containing redundant parts of the images. Following the cropping operation, a further first sub-sequence DS1' is obtained.

For example, the cropping operation allows to eliminate text words or wording placed, usually, in correspondence to the border of each image and carrying the data of the patient. In this way, the images of the further first sub-sequence DS1' are made anonymous. Furthermore, it is possible to eliminate from the ultrasonographic images black portions of the images, which, as known to those skilled in the art, do not contain any information.

It is observed that following the cropping operation the dimensions of each image of the sub-sequence DS1' are reduced in comparison to those of the corresponding images of the sub-sequence DS1. Consequently, the resolution of each image is reduced without reducing the quality of the cropped image.

In addition, the processing phase foresees an image compression of the further sub-sequence DS1', by means of an algorithm or compression codec implemented by the compression block 25 generating a first sub-sequence of compressed images DS1''.

Preferably, the compression codec used is in accordance with a standard MPEG-4 (Motion Picture Expert Group-4) and permits to compress, on average, a stream of data from 52 Mbyte/sec to 152 kByte/sec, i.e. with a compression ratio of about 1000 to 1.

In particular, it is observed that MPEG-4 is a lossy type compression standard, i.e. with such codec the compressed images lose, once decompressed, a percentage of the information contained in the original images before the compression.

For this reason, for the purpose of the invention, it was necessary to validate such standard in the medical field. In other words, it was verified that the same sequence of images before the compression and after being compressed and decompressed, were indistinguishable from each other by human assessment for diagnostic purposes.

The first sub-sequence DS1'' of compressed images with the MPEG-4 codec is memorized locally in the notebook 8, specifically in the mass memory 22.

It is observed that the processing and the memorization of the first sub-sequence DS1 are carried out during a second time interval T2 consecutive to the first interval T1 of pick up of such sub-sequence from the stream of the digital images DS.

Advantageously, it is presumed that such second time interval T2 is sufficiently longer than the time interval that elapses between one image and the next of the image stream.

In particular, the duration of the second time interval T2 depends, mostly, on the duration of the compression operation of the images and varies with the compression codec MPEG-4 used. For example, using known compression codecs, the duration of the second time interval can vary from three to five seconds.

During the second time interval T2 the microprocessor 21 does not acquire any further incoming sub-sequence of images DS inputted to the notebook 8. In other words, the images of the sequence successive to those of the first sub-sequence DS1 are not processed, i.e. they are lost.

Typically, each compression codec MPEG-4 foresees a multiplicity of compression of quality degrees of the images of the sub-sequence DS1'. To each of these degrees corresponds a quality of the images declared by the manufacturer of the algorithm. To the lowest degree of compression (i.e. maximum quality declared by the manufacturer of the algorithm) corresponds a higher quality of the compressed images, while such quality diminishes as the degree of compression increases. For example, utilizing the lowest degree of compression (which corresponds to a 100% quality) the first sub-sequence compressed DS1'' has a dimension of 1,835 Mbyte, utilizing an intermediate compression degree (for example a quality of 65%) the dimension of the sub-sequence is reduced to 251 kByte and at 40% of quality to 119 kByte.

After a time T equal to the sum of the first T1 and the second T2 time intervals, the microprocessor 21 is informed of the occurred memorization of the first compressed sub-sequence DS1'' in the memory 22 and that it is capable of repeating automatically the described elaboration for a second sub-sequence of images. In other words, the microprocessor 21 picks up from the image sequence DS a second sub-sequence DS2, without any intervention by the external operator, but operating following the pre-established instructions of the software client SWCL and memorized in the mass memory 22. On such sub-sequence DS2 the precedent processing from the blocks 23-25 is carried out.

The compressed images of such second sub-sequence DS2 are memorized in the memory 22 after those of the first sub-sequence DS1.

The successive sub-sequences of the image sequence DS are picked up analogously, i.e. automatically, until the end of the echocardiographic session.

In other words, the method of invention foresees that sub-sequences of the digital image stream DS (which are spaced out among them by the second time interval T2) are picked up, processed and memorized, carrying out, therefore, an automated clinical compression.

It is observed that the automation of the clinical compression is advantageous inasmuch as the operator has only to follow the echocardiographic exam moving the probe 3, without the burden of controlling with commands sent to the notebook 8 the acquisition of each single sub-sequence.

This allows the operator to perform in the best possible way the echocardiographic examination.

Advantageously, the operator interfaces with the software client SWCL through a configuration block 27. In particular, the operator can define and modify parameters of the echocardiographic session by acting on the procedures specified by such block 27.

For example, the main modifiable parameters are the duration of the first time interval T1, the cropping degree of the picked up images, the type of codec used in the session and the compression degree of the sub-sequence for the chosen video codec. Other modifiable parameters through the configuration block 27 concern the inclusion or exclusion of the audio during the compression, the selection of the input video device to the notebook 8 and the video input format selection.

Furthermore, the software client SWCL includes a functionality of inserting the alphanumeric data relative to the patient (for example, name, surname, age, etc.), of the data relative to the echocardiographic examination (e.g. an identity code of the echocardiographic session, the duration of the session, the name of the operator, the type of examination carried out, etc.), and of data relative to the medical report (comments and conclusions relative to the diagnostic images). Such functionality is specified by the data insertion block 28 and the alphanumeric data inserted generate a plurality of digital data indicated in figure 2 with the reference DAT.

Advantageously, the software client SWCL also includes a playback functionality of a compressed sub-sequence which has been memorized in the notebook 8. Therefore, during the echocardiographic session the operator can activate such functionality to visualize on the second screen 9 the memorized sub-sequences with the aim to control or compare them.

In an on-line modality, i.e. in the case in which a remote connection has been established between the notebook 8 and the server 10, the sub-sequences located in the mass memory 22 are transferred on the first line 11 and subsequently to the server 10 after having been memorized, i.e. the sub-sequences are transferred in (First In First Out) FIFO modality. In particular, the digital data corresponding to the images of each sub-sequence are organized in packets and are transmitted in accordance to a transmission protocol known as, for example, the FTP protocol (File Transfers Protocol). Such transmission of the compressed sub-sequences is carried out by the specified operations of the transmission block 26.

Analogously, the digital data DAT relative to the patient and the echocardiographic examination are sent to the server 10 as text files through the same transmission protocol. For example, the text file of the data DAT has a dimension of about 2 kbit.

It is noted that the codec MPEG-4 carries out a compression of the digital data associated to the sub-sequences to be transmitted which is high enough to allow the use of a first line 11 as a narrow transmission band.

Furthermore, it is observed that each compressed sub-sequence DS1'' sent to the server 10 arrives at destination after a variable time according to the speed of transmission, thus of the transmission band, offered by the transmission system used.

For example, if the notebook 8 is connected to server 10 through a 56 kbit/sec modem and a telephone duplex line, the first compressed sub-sequence DS1'' of 251 kByte (compression quality degree of 65%) is transmitted to the server 10 in about thirty-five seconds.

Analogously about thirty seconds are needed to transmit the same sub-sequence DS1'' if the notebook 8 is connected to server 10 through a wire-less data transmission/reception board via Internet of the UMTS type. In fact, such UMTS board guarantees an upload transmission speed of approximately 64 kbit/sec.

Advantageously, if the notebook 8 is connected to the server 10 through an ISDN telephone line at 128 kbit/sec (i.e. with two channels of 64 kbit/sec each) the second sub-sequence DS1'' of 251 kByte is transmitted in about fifteen seconds, while using an ASDL line at 256 kbit/sec the sub-sequence is sent in seven seconds.

Furthermore, if the notebook 8 and the server 10 are connected by a dedicated LAN line having a data transmission speed ranging a few Mbit/sec the transmission of the sub-sequence DS1'' is substantially instantaneous.

In correspondence to the server 10, the compressed sub-sequence DS1'' sent from the notebook 8 is decompressed and played back in accordance to operations specified in the program blocks 31 and 32 of the software server SWSV. In this way, the sub-sequence DS1'' is viewable by the user connected to the server 10 through a further processing apparatus 12.

Furthermore, the compressed sub-sequence DS1'' and all the successive compressed sub-sequences of the same echocardiographic session sent to the notebook 8 are memorized in the further mass memory of the server 10. In particular, such compressed image sub-sequences are inserted in a consultable digital image data base.

Analogously, the DAT text file received from the notebook 8 is memorized in the server 10 and is thus associated to the sub-sequence of images of the corresponding session. Such text file is inserted in an further alphanumerical data base containing the data relative to the patient and the data relative to the echocardiographic examination.

In particular, the data bases of the server 10 can be consulted by more than one user (indicated with reference CL in figure 2B) simultaneously. Furthermore the content of such data bases can be sent via fax, printed or written on a CD-ROM.

In accordance to the previous numeric examples, if an ADSL line is used and the necessary time for the decompression of the received sub-sequences is disregarded, the user connected to the server 10 can observe, for example, a sub-sequence with a T1 duration, approximately every seven seconds.

In this way, the user is able to follow an echocardiographic session almost in real time.

Advantageously, the user can provide a remote consultation to the operator who is carrying out the echocardiographic examination while viewing the received sub-sequences. In particular, the user can communicate (e.g. by telephone) to the operator his own impressions on the course of the examination and suggest the opportune movements of the probe 3 in order to optimize the echographic investigation. In this way, it is not necessary that the operator be a medical doctor, but he could simply be an echographic technician.

Furthermore, the method of invention can foresee an audio message exchange as digital signals between the operator and the user to allow them to communicate in real time. In particular, if an ADSL line is used it is possible to reserve a part of the transmission band for the transmission of such digital audio messages.

In an off-line modality, i.e. without remote connection between the notebook 8 and the server 10, the sub-sequences of the whole echocardiographic session are all memorized locally in the mass memory 22 of the notebook 8 in order to be transmitted at a subsequent time.

It is observed that the processing carried out in the notebook 8 occurs independently from the subsequent transmission of the compressed sub-sequences. Therefore, if the remote connection between the notebook 8 and the server 10 should be interrupted, the whole echocardiographic session would be, however, completed and the results of the same (memorized in the notebook 8) would be sent to the server 10 as soon as a remote connection is re-established.

In addition to the "automatic" modality described above, it is possible to foresee that the notebook 8 allows the selection of a "manual" functioning modality by which the operator can decide which sub-sequences of the whole echographic session are most convenient to be processed. In this way the segmentation procedure of the stream of images known as "clinical compression" is realized. In other words, after a pick up of a first sub-sequence of the stream of images (following the pressing of a key of the key board of notebook 8), its subsequent compression and memorization, the processing is interrupted and the operator can decide after how much time (pressing the same key) to pick up the successive sub-sequence.

As already said, the method and the invention system result particularly advantageous thanks to the implementation of the clinical compression in an automatic way. Furthermore, the use of the MPEG-4 compression codec allows an effective implementation of the transmission of the sub-sequences of the echocardiographic images towards a remote server (such as server 10) allowing to realize the tele-consultation at low costs. In fact, the MPEG-4 codec allows the transmission of the sub-sequence on narrow band transmission lines, for example, ISDN or ADSL lines.

On the contrary, the conventional technique that uses the MPEG-2 codec would allow the transmission of the sub-sequences with dedicated broad band transmission lines which, as known, are very expensive.

Furthermore, differently from video clips compressed with an MPEG-4 codec, the video clips compressed with an MPEG-2 codec need large storage spaces and have to be transferred to a remote station, using a LAN line, for both archiving and reporting purposes.

Furthermore, during the carrying out of the echocardiographic examination with a conventional technique, the remote transmission of the acquired video-clip is not possible inasmuch as these latter are temporarily memorized on the echographic apparatus which is not equipped for remote transmission.

Instead, during the examination, the video clips could be sent to a server placed in a different location from that in which is placed the operator who, therefore, cannot simultaneously manage a remote transmission.

It is observed that the use of MPEG-4 for echographic video clips or generally for clinical image types is totally new and innovative.

Finally, it is relevant to observe that the method of the invention is also particularly suitable to be used for a clinical consultation service, for methodological updating (clinical and/or scientific) and for remote teaching.

Obviously, those skilled in the art, in order to satisfy contingent requirements and specifications, could bring further modifications and variants to the system and to the digital images processing method of the present invention, all being contemplated within the scope of protection of the invention, such as defined by the following claims.

## Claims

1. A method for processing by means of a processing unit (21) a sequence (DS) of digital ultrasonic images obtained, from a medical ultrasonographic equipment (1), including the phases of:
a) acquiring a first sub-sequence (DS1) of ultrasonic images of said sequence having a modifiable duration equal to a first time interval (T1);
b) processing the first sub-sequence of a plurality of ultrasonic images to obtain a first plurality of digital data (DS1") ;
c) memorizing the first plurality of data in memorizing means (22); the phases of processing and memorization being carried out within a second time interval (T2) consecutive to the first time interval and during which the acquisition of further sub-sequences is inhibited;
**characterized in that** the processing unit (21) commands, after a time (T) substantially equal to the sum of the first and second interval and automatically, at least one repetition of the phases a)- c), memorizing at least a second plurality of digital data relative to a second sub-sequence (DS2) of ultrasonic images.

2. A method in accordance with claim 1, in which the phase of processing the first sub-sequence (DS1) includes the phases of:
b1) eliminating image portions from the images of the first acquired sub-sequence to obtain a further sub-sequence of images (DS1');
b2) compressing the further sub-sequence of images, to obtain said first plurality of digital data (DS1").

3. A method in accordance with claim 1, including a phase of making available the sequence of digital ultrasonic images (DS) to be processed to a processing apparatus (8) including said processing unit (21) and said memorizing means (22).

4. A method in accordance with claim 3, including a phase of generating the sequence of digital ultrasonic images (DS) by manipulation, by an operator, of a probe (3) of the medical ultrasonographic equipment (1) on a living human body.

5. A method in accordance with claim 3, further comprising a phase of transmitting, in an on-line modality, the first plurality of digital data (DS1'') memorized in the storage means (22) to a service processor (10) connected to said processing apparatus (8) to create a data base of digital images at the level of the service processor.

6. A method in accordance with claim 5, in which the phase of transmitting the first plurality of digital data (DS1'') includes a phase of sending said data on a first convection line (11) between the processing apparatus (8) and the service processor (10).

7. A method in accordance with claim 6, in which said first line (11) is a fixed network broad band transmission line, preferably of the LAN or VPN type.

8. A method in accordance with claim 6, in which said first line (11) is a narrow band transmission line, which, preferably is an ISDN or ADSL telephone line.

9. A method in accordance with claim 6, in which said first line (11) is a GPRS or UMTS radio mobile transmission line.

10. A method in accordance with claim 1, in which the processing apparatus (8) is re-configurable to modify parameters relative to the phases of acquisition and processing.

11. A method in accordance with claim 3, including a phase of inserting alphanumeric data which identify a living human being and/or a correspondent echographic examination in order to generate a further plurality of digital, data (DAT) to be transmitted to the service processor (10) using a non compressed modality.

12. A method in accordance with claims 3 and 4, including a phase of visualizing on a screen (9) of the processing apparatus (8) the memorized sub-sequences (DS1, DS2) of images in order to allow n operator to control or compare the same sub-sequences.

13. A method in accordance with claims 2 or 8, in which said phase of compression of the first sub-sequence (DS1) of images includes a phase of implementing a MPEG-4 compression algorithm.

14. A method in accordance with claims 5 and 11, including the further phases of:
- memorizing, in correspondence of the service processor (10), the first compressed sub-sequence (DS1") and the further plurality of data (DAT) received in further memorizing means;
- decompressing the sub-sequence to make it accessible, substantially, in real time to a user connected to the service processor through a second line of connection (13).

15. A method in accordance with claims 4 and 13 including, furthermore, a phase to establish a communication between the user and the operator relative to the modality of probe manipulation.

16. A, method in accordance with claim 5, including furthermore a phase of accessing through a telecommunication network said data base of digital images.

17. Processing apparatus (8) of a sequence (DS) of digital ultrasonic images, including:
- a processing unit (21) to acquire a first sub-sequence (DS1) of images of said sequence and to process it in order to obtain a first plurality of digital data (DS1"),
- memorizing means (22) to memorize the plurality of digital data,
**characterized in that** it is carried out in such a way to operate as a processing apparatus of the defined method by at least one of the previous claims.

18. Digital ultrasonic images processing and transmission system (100), including:
- a processing apparatus (8) of a sequence (DS) of digital ultrasonic images adapted to transmit on a connection line (11) a first plurality of digital data obtained starting from said sequence;
- a service processor (10) connected to said line to receive the first plurality of data and to allow the visualization of corresponding images,
**characterized in that** said system is accomplished in such a way to operate in accordance to the processing method defined in at least one of the claims from 1 to 16.

19. System (100) according to claim 18, comprising furthermore an equipment (1) to acquire the sequence of ultrasonic images (DS) relative to a clinical examination.

## Patentansprüche

1. Verfahren zum Verarbeiten einer Folge (DS) von digitalen Ultraschallbildern, die von einer medizinischen Ultraschallanlage (1) erhalten wurden, mittels einer Prozessoreinheit (21) umfassend die Phasen:
a) Beschaffen einer ersten Unterfolge (DS1) von Ultraschallbildern der Folge, die eine veränderbare Dauer gleich einem ersten Zeitintervall (T1) hat;
b) Verarbeiten der ersten Unterfolge von mehreren Ultraschallbildern, um eine erste Vielzahl digitaler Daten (DS1") zu erhalten;
c) Speichern der ersten Vielzahl Daten in Speichereinrichtungen (22); wobei die Phasen der Verarbeitung und der Speicherung innerhalb eines zweiten Zeitintervalls (T2) im Anschluss an das erste Zeitintervall ausgeführt werden und währenddessen die Beschaffung weiterer Unterfolgen gesperrt ist;
**dadurch gekennzeichnet, dass** die Prozessoreinheit (21) nach einer Zeit (T), die im Wesentlichen gleich der Summe aus dem ersten und dem zweiten Intervall ist, und automatisch wenigstens eine Wiederholung der Phasen a) bis c) anordnet, wobei wenigstens eine zweite Vielzahl digitaler Daten, die sich auf eine zweite Unterfolge (DS2) von Ultraschallbildern bezieht, gespeichert wird.

2. Verfahren nach Anspruch 1, bei dem die Phase der Verarbeitung der ersten Unterfolge (DS1) die folgenden Phasen beinhaltet:
b1) Beseitigen von Bildabschnitten aus den Bildern der ersten beschafften Unterfolge, um eine weitere Unterfolge (DS1') von Bildern zu erhalten;
b2) Komprimieren der ersten Unterfolge von Bildern, um die erste Vielzahl digitaler Daten (DS1") zu erhalten.

3. Verfahren nach Anspruch 1, enthaltend eine Phase der Verfügbarmachung der Folge von zu verarbeitenden Ultraschallbildern (DS) für ein Verarbeitungsgerät (8), das die Prozessoreinheit (21) und die Speichereinrichtungen (22) enthält.

4. Verfahren nach Anspruch 3, enthaltend eine Phase der Erzeugung der Folge digitaler Ultraschallbilder (DS) durch Manipulation einer Sonde (3) der medizinischen Ultraschallanlage (1) an einem lebenden menschlichen Körper durch eine Bedienperson.

5. Verfahren nach Anspruch 3, weiterhin enthaltend eine Phase des Sendens, in einem Online-Modus, der ersten Vielzahl in den Speichereinrichtungen (22) gespeicherter digitaler Daten (DS1") an einen Serviceprozessor (10), der mit dem Verarbeitungsgerät (8) verbunden ist, um eine Datenbank aus digitalen Bildern auf Höhe des Serviceprozessors zu erstellen.

6. Verfahren nach Anspruch 5, bei dem die Phase des Sendens der ersten Vielzhal digitaler Daten (DS1") eine Phase des Sendens dieser Daten auf einer ersten Verbindungsleitung (11) zwischen dem Verarbeitungsgerät (8) und dem Serviceprozessor (10) enthält.

7. Verfahren nach Anspruch 6, bei dem die erste Leitung (11) eine Festnetzwerk-Breitbandübertragungsleitung, vorzugsweise vom LAN- oder VPN-Typ, ist.

8. Verfahren nach Anspruch 6, bei dem die erste Leitung (11) eine Schmalbandübertragungsleitung ist, die vorzugsweise eine ISDN- oder ADSL-Telefonleitung ist.

9. Verfahren nach Anspruch 6, bei dem die erste Leitung (11) eine GPRS- oder UMTS-Mobilfunksendeleitung ist.

10. Verfahren nach Anspruch 1, bei dem das Verarbeitungsgerät (8) umkonfigurierbar ist, um Parameter, die sich auf die Phasen der Beschaffung und der Verarbeitung beziehen, zu verändern.

11. Verfahren nach Anspruch 3, enthaltend eine Phase des Einfügens alphanumerischer Daten, die ein lebendes menschliches Wesen und/oder eine entsprechende echografische Prüfung identifizieren, um eine weitere Vielzahl digitaler Daten (DAT) zu erzeugen, die an den Serviceprozessor (10) unter Verwendung einer nicht komprimierten Art und Weise zu senden sind.

12. Verfahren nach den Ansprüchen 3 und 4, enthaltend eine Phase des Anzeigens der gespeicherten Unterfolgen (DS1, DS2) von Bildern auf einem Bildschirm (9) des Verarbeitungsgeräts (8), um es einer Bedienperson zu ermöglichen, die gleichen Unterfolgen zu kontrollieren oder zu vergleichen.

13. Verfahren nach Anspruch 2 oder 8, bei dem die Phase des Komprimierens der ersten Unterfolge (DS1) von Bildern eine Phase des Ausführens eines MPEG-4-Kompressions-algorithmus enthält.

14. Verfahren nach den Ansprüchen 5 und 11, enthaltend die weiteren Phasen:
- Speichern, in Korrespondenz mit dem Serviceprozessor (10), der ersten komprimierten Unterfolge (DS1 ") und der weiteren Vielzahl von Daten (DAT), die in weiteren Speichereinrichtungen empfangen werden;
- Dekomprimieren der Unterfolge, um sie im Wesentlichen in Echtzeit für einen Benutzer zugänglich zu machen, der mit dem Serviceprozessor über eine zweite Verbindungsleitung (13) verbunden ist.

15. Verfahren nach den Ansprüchen 4 und 13, enthaltend weiterhin eine Phase zur Einrichtung einer Kommunikation zwischen dem Benutzer und der Bedienperson bezüglich der Art und Weise der Sondenmanipulation.

16. Verfahren nach Anspruch 5, enthaltend außerdem eine Phase des Zugriffs zu der Datenbank aus digitalen Bildern über ein Telekommunikationsnetzwerk.

17. Verarbeitungsgerät (8) für eine Folge (DS) von digitalen Ultraschallbildern, enthaltend:
eine Prozessoreinheit (21) zur Beschaffung einer ersten Unterfolge (DS1) von Bildern der Folge und zur Verarbeitung derselben, um eine erste Vielzahl digitaler Daten (DS1") zu erhalten,
Speichereinrichtungen (22) zur Speicherung der Vielzahl digitaler Daten,
**dadurch gekennzeichnet, dass** es in solcher Weise ausgeführt ist, dass es als ein Ausführungsgerät für das von wenigstens einem der vorhergehenden Ansprüche angegebene Verfahren arbeitet.

18. System (100) zum Verarbeiten und Senden von digitalen Ultraschallbildern, enthaltend:
- ein Gerät (8) zur Verarbeitung einer Folge (DS) von digitalen Ultraschallbildern, das zum Senden einer ersten Vielzahl digitaler Daten, die ausgehend von der Folge erhalten werden, auf einer Verbindungsleitung (11) eingerichtet ist;
einen Serviceprozessor (10), der mit der Leitung verbunden ist, um die erste Vielzahl von Daten zu empfangen und die Anzeige entsprechender Bilder zu ermöglichen,
**dadurch gekennzeichnet, dass** das System derart ausgeführt ist, dass es in Übereinstimmung mit dem Verarbeitungsverfahren arbeitet, das in wenigstens einem der Ansprüche 1 bis 16 angegeben ist.

19. System (100) nach Anspruch 18, enthaltend weiterhin eine Einrichtung (1) zur Beschaffung der Folge (DS) von Ultraschallbildern, die sich auf eine klinische Prüfung beziehen.

## Revendications

1. Procédé de traitement, à l'aide d'une unité de traitement (21), d'une séquence (DS) d'images ultrasoniques numériques obtenues d'un équipement médical ultrasonographique (1), comprenant les étapes suivantes :
a) acquérir une première sous-séquence (DS1) d'images ultrasoniques de ladite séquence ayant une durée modifiable égale à un premier intervalle de temps (T1) ;
b) traiter la première sous-séquence comprenant plusieurs images ultrasoniques afin d'obtenir une première pluralité de données numériques (DS1") ;
c) mémoriser la première pluralité de données dans un moyen de mémorisation (22) ; les phases de traitement et de mémorisation s'effectuant au cours d'un second intervalle de temps (T2) consécutif au premier intervalle de temps et pendant lequel l'acquisition d'une autre sous-séquence est empêchée ;
**caractérisé en ce que** l'unité de traitement (21) ordonne automatiquement, après un temps (T) essentiellement égal à la somme des premier et second intervalles, au moins une répétition des phases a) ― c), en mémorisant au moins une seconde pluralité de données numériques concernant une seconde sous-séquence (DS2) d'images ultrasoniques.

2. Procédé selon la revendication 1, dans lequel la phase de traitement de la première sous-séquence (DS1) comprend les phases suivantes :
b1) éliminer des parties d'image des images de la première sous-séquence acquise afin d'obtenir une sous-séquence supplémentaire d'images (DS1') ;
b2) comprimer la sous-séquence supplémentaire d'images afin d'obtenir ladite première pluralité de données numériques (DS1").

3. Procédé selon la revendication 1, comprenant une phase consistant à rendre la séquence d'images ultrasoniques numériques (DS) à traiter disponible pour un appareil de traitement (8) comprenant ladite unité de traitement (21) et ledit moyen de mémorisation (22).

4. Procédé selon la revendication 3, comprenant une phase consistant à générer la séquence d'images ultrasoniques numériques (DS) par manipulation, par un opérateur, d'une sonde (3) de l'équipement médical ultrasonographique (1) sur un corps humain vivant.

5. Procédé selon la revendication 3, comprenant en outre une phase de transmission, selon une modalité en ligne, de la première pluralité de données numérique (DS1") mémorisée dans le moyen de stockage (22) vers un processeur de service (10) connecté au dit appareil de traitement (8) afin de créer une base de données d'images numériques au niveau du processeur de service.

6. Procédé selon la revendication 5, dans lequel la phase de transmission de la première pluralité de données numériques (DS1") comprend une phase consistant à envoyer lesdites données sur une première ligne de communication (11) entre l'appareil de traitement (8) et le processeur de service (10).

7. Procédé selon la revendication 6, dans lequel ladite première ligne (11) consiste en une ligne de transmission en bande large d'un réseau fixe, de préférence du type LAN ou VPN.

8. Procédé selon la revendication 6, dans lequel ladite première ligne (11) est une ligne de transmission à bande étroite consistant de préférence en une ligne téléphonique ISDN ou ADSL.

9. Procédé selon la revendication 6, dans lequel ladite première ligne (11) est une ligne de transmission mobile radio GPRS ou UMTS.

10. Procédé selon la revendication 1, dans lequel l'appareil de traitement (8) est reconfigurable afin de modifier les paramètres concernant les phases d'acquisition et le traitement.

11. Procédé selon la revendication 3, comprenant une phase consistant à insérer des données alphanumériques qui identifient un être humain vivant et/ou un examen échographique correspondant afin de générer une autre pluralité de données numériques (DAT) à transmettre au processeur de service (10) en utilisant une modalité de non-compression.

12. Procédé selon les revendications 3 et 4, comprenant une phase consistant à visualiser sur un écran (9) de l'appareil de traitement (8), les sous-séquences mémorisées (DS1, DS2) des images afin de permettre à un opérateur de contrôler ou de comparer ces mêmes sous-séquences.

13. Procédé selon les revendications 2 ou 8, dans lequel ladite phase de compression de la première sous-séquence (DS1) d'images comprend une phase consistant à mettre en oeuvre un algorithme de compression MPEG-4.

14. Procédé selon les revendications 5 et 11, comprenant en outre les phases consistant à :
- mémoriser, en correspondance avec le processeur de service (10), la première sous-séquence compressée (DS1") et l'autre pluralité de données reçues dans un autre moyen de mémorisation ;
- décompresser la sous-séquence afin de la rendre accessible essentiellement en temps réel, à un utilisateur connecté au processeur de service (10) par une seconde ligne de connexion (13).

15. Procédé selon les revendications 4 et 13, comprenant en outre une phase consistant à établir une communication entre l'utilisateur et l'opérateur concernant la modalité de manipulation de la sonde.

16. Procédé selon la revendication 5, comprenant en outre une phase consistant à accéder, par le biais d'un réseau de télécommunication, à la base de données des images numériques.

17. Appareil de traitement (8) d'une séquence (DS) d'images ultrasoniques numériques comprenant :
- une unité de traitement (21) pour acquérir une première sous-séquence (DS1) d'images de ladite séquence et pour la traiter afin d'obtenir une première pluralité de données numériques (DS1") ;
- un moyen de mémorisation (22) afin de mémoriser la pluralité de données numériques ;
**caractérisé en ce qu'**il est réalisé de manière à fonctionner comme un appareil de traitement selon le procédé défini dans l'une au moins des revendications précédentes.

18. Système de traitement et de transmission d'images ultrasoniques numériques (100) comprenant :
- un appareil de traitement (8) d'une séquence (DS) d'images numériques ultrasoniques conçu pour transmettre sur une ligne de connexion (11) une première pluralité d'images numériques obtenues à partir de ladite séquence ;
- un processeur de service (10) connecté à ladite ligne afin de recevoir la première pluralité de données et de permettre la visualisation des images correspondantes ;
**caractérisé en ce que** ledit système est réalisé de manière à fonctionner selon le procédé de traitement défini dans l'une au moins des revendications 1 à 16.

19. Système (100) selon la revendication 18, comprenant en outre un équipement (19 permettant d'acquérir la séquence d'images ultrasoniques (DS) relative à un examen clinique.
